# EUROPEAN PATENT APPLICATION

(11) **EP 2 762 168 A1**
(43) Date of publication of application: **06.08.2014**
(21) Application number: 14153805.8
(22) Date of filing: 04.02.2014
(51) Int. Cl.: A61L 2/07, B01J 3/04

(54) **Autoclave for sterilizing instruments**

(30) Priority: 05.02.2013 IT BO20130050
(71) Applicant: MOCOM S.r.L., 40026 Imola (BO) (IT)
(72) Inventor: BELLOSI, Angelo, 40026 IMOLA (Bologna) (IT)
(74) Representative: Lunati & Mazzoni S.r.L.

(57) **Abstract**

Autoclave (1) for sterilizing medical and/or dental instruments, comprising a sterilization chamber (3), a door (2) for access to sterilization chamber (3) characterized in that in autoclave (1) at least a light source (4) is present for lightening sterilization chamber (3) and/or the area (7) in front of autoclave (1).

## Description

The present invention relates to an autoclave for sterilizing surgical and/or dental instruments, and more particularly to a lighting system for the autoclave. Similar autoclaves are described in patent applications EP-A-0575005, US-A-5223229 and US-A-2004/001783.

In the medical and dental field, sterile instruments must be used in order to prevent patient-to-patient cross-infection. One of the most usual solutions for sterilizing instruments is the use of an autoclave, either a steam autoclave or a chemical vapour autoclave.

Typically, the autoclave finds its place in a sterilizing room, comprising all the necessary apparatuses, like thermo-disinfector, ultrasonic bath, thermal sealer, etc. Due to its shape, typically the autoclave is placed with its rear against a wall, and its access door to the sterilisation chamber faces the centre of the room. The operator has to suitably place the instruments to be sterilized in the autoclave sterilisation chamber, using special trays. After that, the autoclave door is closed and the sterilization cycle can start, introducing steam into the sealed sterilisation chamber. During autoclave loading/unloading operations, the operator generally turns her/his back to the light, which generally is in the centre of the room, projecting a shadow cone towards the autoclave itself. It is worthwhile noting that the instruments to be sterilized are often sharps (burs, lancets, scissors, probes, etc.), and the operator is prone to the risk of lesions that can lead to heavy consequences, if the instruments are contaminated with pathogens causing pathologies like AIDS, hepatitis B or C, tuberculosis, etc.

It is the object of the present invention to improve the safety and the comfort of the operator in the sterilization room, by lighting the work area and the sterilisation chamber of the autoclave.

It is another object of the present invention to improve the human-machine interface using the light source used to lighten the work area also for generating messages on the working status of the autoclave, particularly when the operator is acoustically impaired.

This object is achieved by an apparatus having the features of the independent claim. Advantageous embodiment and refinements are specified in the claims dependent thereon.

According to the present invention, a LED bar is placed over the entry of the autoclave sterilisation chamber; the LED bar is integral with the autoclave and is insulated from both the external environment and the sterilisation chamber itself. The LED bar is placed so that operator's glare is prevented, and is parallel to the autoclave front, so that a homogeneous and diffused illumination of the area in front of the autoclave and of the sterilisation chamber is achieved.

The LED bar lights up when the autoclave door is open, upon command of a microswitch detecting the condition of open/closed door. When the door is open, is nonetheless possible to intervene on the LED bar feeding so as to switch it off or to vary the lighting conditions.

Further advantages and properties of the present invention are disclosed in the following description, in which exemplary embodiments of the present invention are explained in detail based on the drawings:
**Fig. 1** Perspective view of the autoclave on the whole, with closed access door;
**Fig. 2** Frontal perspective view of the autoclave on the whole, with open access door and lighted up light source;
**Fig. 3** Perspective bottom view of an autoclave detail, with open access door.

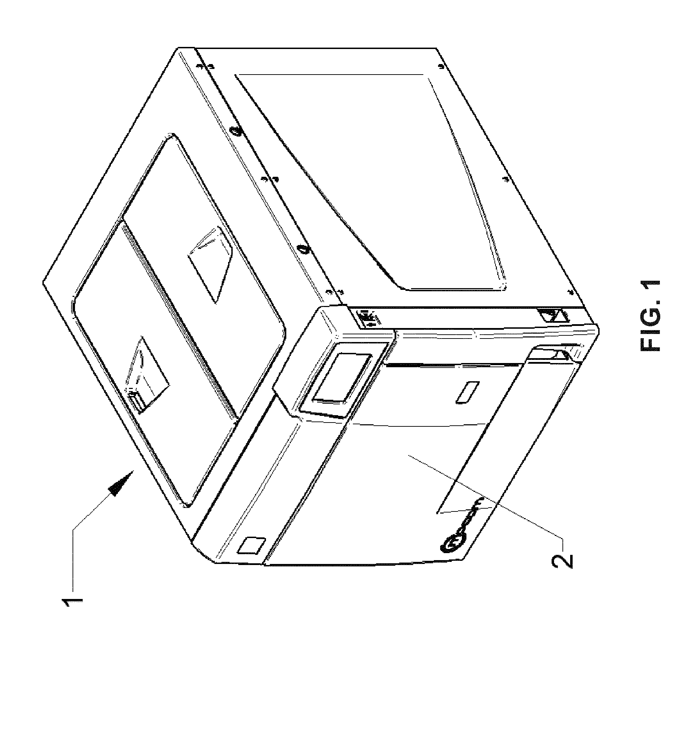

In Figure 1, representing the closed autoclave, 1 indicates an autoclave on the whole; 2 indicates autoclave door placed on the frontal area of the autoclave.

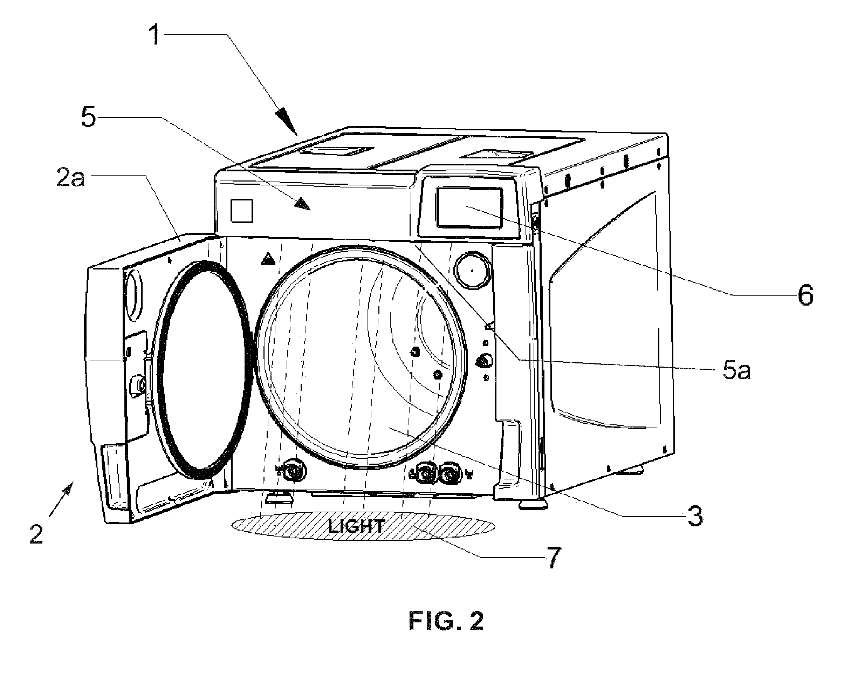

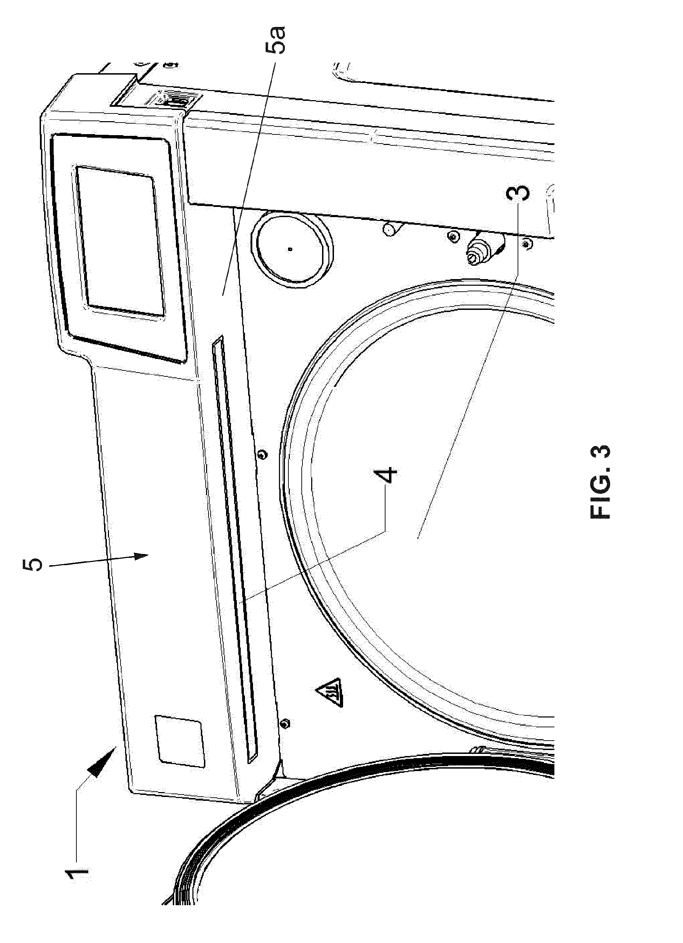

Figure 2 shows the autoclave 1 with open door 2; 3 is the sterilisation chamber. A light source 4, which in the preferred embodiment is a LED bar, which is a plurality of LED lighting aligned on the same bar, is visible in Figure 3. In the preferred embodiment, light source 4 is placed in the upper portion 5, on the frontal area over the entry of sterilisation chamber 3.

More in detail, the door 2 of the autoclave 1 has a thickness preferably of the order of magnitude of centimetres that opportunely defines a top face 2a, perpendicular to the face of prevailing extension of the same door 2 and, in use, pointing upwards and preferably horizontal.

The upper part of the upper portion 5 therefore presents a lower face 5a, facing the upper face 2a of the door 2, and then also preferably, in use, horizontal and pointing downwards.

The source 4 is disposed on and faces the lower face 5a.

In Figure 2 light rays emitted from light source 4 are represented with hatching; they form a lighted area 7 in front of the autoclave, indicated with LIGHT. The LEDs in the bar are chosen with an angle of the light beam suitable to lighten the area in front of the autoclave and, at least partially, the inside of the sterilisation chamber 3. The drawbacks due to the fact that, as previously described, the operator usually projects a shadow cone towards the autoclave are therefore overcome.

Light source 4 is normally switched off when door 2 is closed, and is lighted up when door 2 is opened. The lighting up of light source 4 is controlled by a microswitch detecting the position of door 2. The signal is received by an electronic board controlling light source 4 providing current through the relative electrical circuit.

Light source 4 must be insulated and protected from both heat and steam which are produced during sterilization cycle, which could lead to damage the light source 4 itself when the door 2 is opened. Insulation is realized by placing the light source 4 in a steam-proof containing element. The outward-facing portion of containing element is translucent and acts as a diffuser, so that light 7 is homogeneous.

Light source 4 can remain lighted up for all the time during which door 2 remains open. In an alternative embodiment, the lighted condition can be programmed in different ways, acting on turn off delay. For instance, in an embodiment the turn off can be programmed after a pre-defined time starting from the opening of door 2. Alternatively, light source 4 can lighten up when door 2 is opened, and the operator can manually act on the lighted/unlighted condition using touch screen display 6 or a touch (not shown) present on the front of autoclave 1.

In a further embodiment, the turn on and turn off of light source 4 can occur in a progressive way, giving rise to a light ramp.

In a further embodiment, light source 4 can be used, in addition to lighting the area 7 in front of autoclave 1, also for providing visual signals in addition to, or in alternative to, acoustic signals normally present in autoclaves. For instance, light pulses can be modulated to indicate the correct conclusion of the sterilization cycle end, or a danger due to high temperature in the sterilisation chamber 3.

Using a light source 4 comprising coloured LEDs, or other types of coloured light sources, it is possible to increase the number of visual signals that can be generated by the autoclave, improving the interpretation of the autoclave condition by the operator. For instance, the lighting up of a green LED might signal the correct end of the sterilization cycle, while the lighting of a red LED might signal an anomaly occurred during the cycle, which therefore was not successful.

The possibility of transmitting visual messages concerning the autoclave operating status in addition or in alternative to acoustic signals becomes very interesting in case the operator is acoustically impaired.

In a further embodiment, LEDs can be maintained lighted up even when door 2 is closed, so that a blade of light filters through the space between upper portion 5 and door 2. This might have a further signal function: for instance a red LED might light up and remain lighted during the sterilization cycle when an anomaly occurs, so that the operator can become aware of the problem already during the cycle performance, and take opportune measures.

## Claims

1. Autoclave (1) for sterilizing medical and/or dental instruments, comprising a sterilization chamber (3), a door (2) for access to sterilization chamber (3) door placed on the frontal area of the autoclave (1), the door (2) having a thickness which defines a top face (2a), substantially perpendicular to the main extension face of the same door (2) and, in use, pointing upwards, the frontal area of the autoclave (1) furthermore comprising an upper portion (5) including a lower face (5a), facing the upper face (2a) of the door (2) and, in use, pointing downwards, in autoclave (1) at least a light source (4) is present for lightening sterilization chamber (3) and/or the area (7) in front of autoclave (1), **characterized in that** the light source (4) is placed on said lower face (5a).

2. Autoclave according to claim 1, wherein light source (4) lights up when the door (2) is opened.

3. Autoclave according to claim 1, wherein light source (4) comprises at least a Light Emitting Diode (LED).

4. Autoclave according to claim 1, wherein the at least one LED is a white light LED.

5. Autoclave according to claim 1, wherein light source (4) can be manually controlled through a control placed on the upper portion (5) of autoclave (1).

6. Autoclave according to claim 1, wherein light source (4) is progressively controlled so as to get a light ramp.

7. Autoclave according to claim 1, wherein light source (4) is controlled so as to automatically switch off after a pre-defined time from its start.

8. Autoclave according to claim 1, wherein light source (4) is powered up also with closed door (2) to transmit visual signals for signalling working conditions and/or anomalies.

9. Autoclave according to claim 8, wherein light source (4) can emit both white light and coloured light.

10. Autoclave according to any of the preceding claims, wherein light source (4) is insulated both from external environment and sterilization chamber (3).
